(19) Europäisches Patentamt
European Patent Office
Office européen des brevets

(11) Publication number: 0 331 315 A2

(12) # EUROPEAN PATENT APPLICATION

(21) Application number: 89301522.2

(51) Int. Cl.4: C07D 211/90

(22) Date of filing: 16.02.89

Claims for the following Contracting State: ES.

(30) Priority: 27.02.88 GB 8804630

(43) Date of publication of application: 06.09.89 Bulletin 89/36

(84) Designated Contracting States: AT BE CH DE ES FR GB GR IT LI LU NL SE

(71) Applicant: **Pfizer Limited**
**Ramsgate Road**
**Sandwich Kent CT13 9NJ(GB)**

(72) Inventor: **Arrowsmith, John Edmund, Dr.**
**340 Dover Road**
**Upper Walmer Deal Kent(GB)**

(74) Representative: **Wood, David John**
**Pfizer Limited Ramsgate Road**
**Sandwich Kent CT13 9NJ(GB)**

(54) **Preparation of R- and S-amlodipine.**

(57) A method of preparing the R- or S- form of the calcium antagonist amlodipine, or of an acid addition salt thereof, which comprises

(i) forming a salt of R,S-2-(2-azidoethoxymethyl)-4-(2-chlorophenyl)-5-methoxycarbonyl-6-methyl-1,4-dihydropyridine-3-carboxylic acid with cinchonidine and separating said salt into its diastereomeric forms by fractional crystallisation;

(ii) converting said separated salts into the free acids by acidification;

(iii)esterifying the free carboxy groups to ethoxycarbonyl; and

(iv) reducing the azido groups to amino, thus producing separated R-(-)- and S-(+)- amlodipine, and isolating the separated forms of amlodipine as such or in acid addition salt form.

The invention also includes the novel intermediates obtained by the above process.



Xerox Copy Centre

## PREPARATION OF R- AND S-AMLODIPINE

Amlodipine (1) [see sheet of formula drawings hereinafter] is a long-acting dihydropyridine calcium channel blocker currently in late stage clinical development for the treatment of angina and hypertension. Amlodipine is a racemic compound due to its assymmetry at position 4 of the dihydropyridine ring. The calcium channel blocking activity resides predominantly in the R-(-) isomer, the S-(+) form being significantly less potent in vitro. Arrowsmith et. al. in J. Med. Chem., 1986, 29, 1696 have described the preparation of the two enantiomers of amlodipine via the separation of the diastereotopic esters (2), but there is a need for an improved method.

The present improved method of the invention is to fractionally crystallise the cinchonidine salts of the previously-described acid (3), i.e. the salts of cinchonidine formed by reaction with racemic 2-(2-azidoethoxymethyl)-4-(2-chlorophenyl)-5-methoxycarbonyl-6-methyl-l,4-dihydropyridine-3-carboxylic acid. The separated diastereomeric cinchonidine salts can then be converted to the R- and S- forms of amlodipine, or an acid addition salt thereof (e.g. a maleate), conventionally, e.g. by acidification of the separated salts, esterification of the resulting free 3-carboxy group to convert it to ethoxycarbonyl, and reduction of the azido group to amino.

The cinchonidine salts (4) and (5), and the acids (6) and (7), also form a part of the invention.

A preferred technique for the separation is illustrated schematically as follows:-

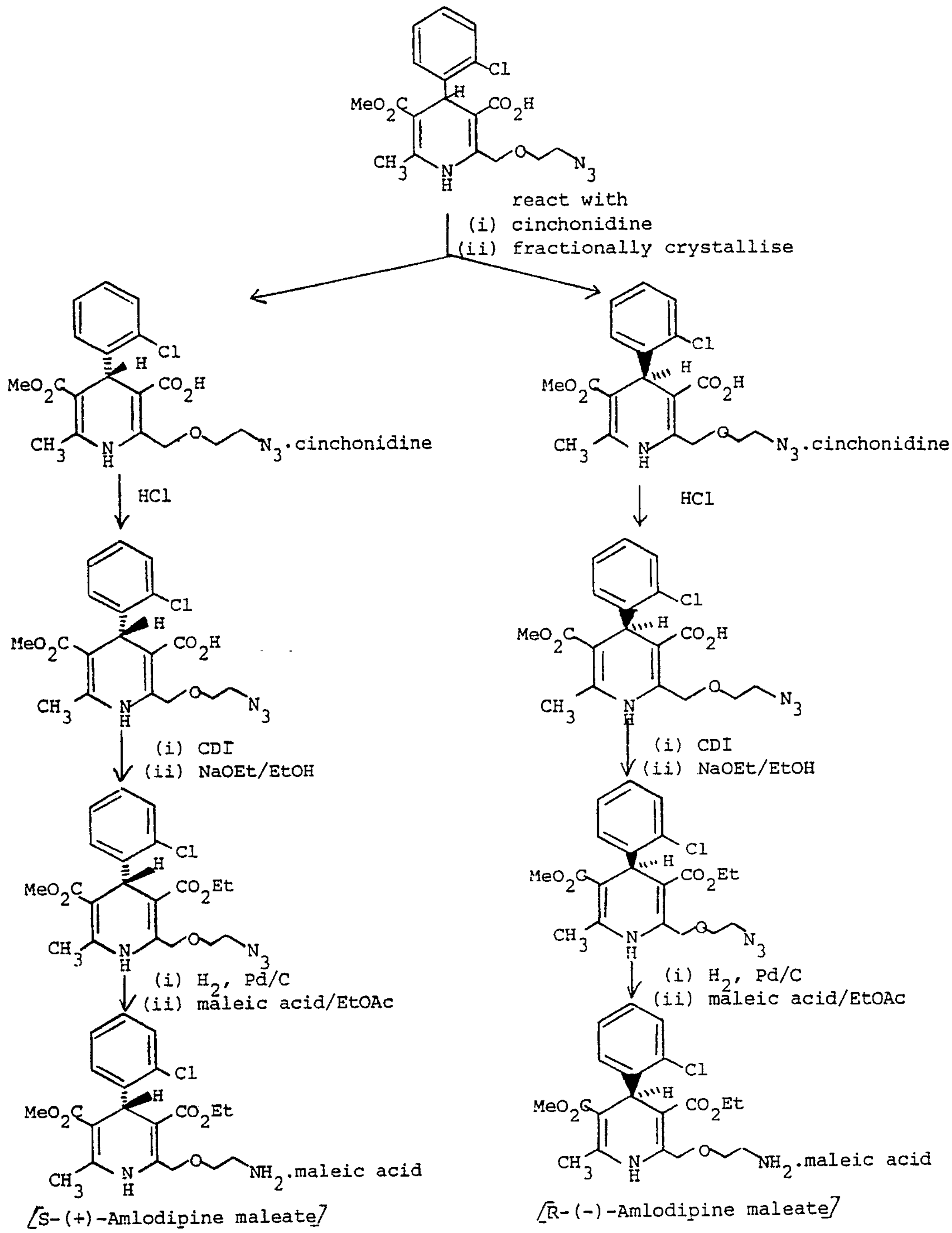


[S-(+)-Amlodipine maleate] [R-(-)-Amlodipine maleate]

[CDI = carbonyldiimidazole].

The racemic acid (3) is preferably converted to its cinchonidine salts in methanolic solution and, upon dilution with water and standing at room temperature, a crystalline precipitate is formed which can be subsequently recrystallised to constant rotation to give the diastereomerically pure cinchonidine salt (4). The mother liquors from the original crystallisation can then be reduced in volume and stirred at room temperature, e.g. overnight, to afford a fine precipitate which can be recrystallised to give the diastereomerically pure cinchonidine salt (5). Conversion of these salts to R-(-) and S-(+) amlodipine is then typically carried out as follows. The cinchonidine salt (4) is partitioned between ethyl acetate and dilute hydrochloric acid to liberate the acid (+) (6). The acid (6) is then esterified in near-quantitative yield by forming an imidazolide (using carbonyldiimidazole) and decomposing this with ethanolic sodium ethoxide to

give (8). The azido group in (8) can then be cleanly reduced to amino by catalytic hydrogenation, giving S-(+) amlodipine, which is most conveniently isolated as an acid addition salt, e.g. as the maleate (10). Similarly (5) can be converted to R-(-) amlodipine maleate (11).

The following Examples illustrate the invention. The structure of all the compounds were confirmed by microanalysis and $^1$H NMR. The $^1$H NMR were routinely recorded on a General Electrics QE-300 and the enantiomeric purities were determined on a Bruker AM400.

## EXAMPLE

### (A) Resolution of the cinchonidine salt of racemic 2-(2-azidoethoxymethyl)-4-(2-chlorophenyl)-5-methoxycarbonyl-6-methyl-1,4-dihydropyridine-3-carboxylic acid (3)

2-(2-Azidoethoxymethyl)-4-(2-chlorophenyl)-5-methoxycarbonyl-6-methyl-1,4-dihydropyridine-3-carboxylic acid (3) (4.64 g, 11.4 mmol) (see J. Med. Chem., 1986, vol. 29, no. 9, page 1701) and cinchonidine (3.36 g, 11.4 mmol) were dissolved in hot methanol, evaporated to dryness and the resulting salt was taken up in hot methanol (150 ml) and diluted with water (25 ml). After cooling to room temperature, the solution was allowed to stand in a refridgerator for 3 days and the resultant crystalline precipitate (3.1 g) was collected by filtration and recrystallised twice from 10% aqueous methanol (15 ml) to afford the cinchonidine salt (4), yield 2.27 g (28%), m.p. 170-171°C, $[\alpha]_D^{25}$ = -78.9° (c = 1.06%, methanol). [The analysis figures are given in a separate Table.]

The mother liquor from the original crystallisation was evaporated to dryness and the residue taken up in hot methanol (70 ml) and diluted with water (20 ml). After cooling to room temperature, the solution was stirred overnight and the resultant powdery precipitate (1.76 g) was collected by filtration and recrystallised twice from 10% aqueous methanol (10 ml) to afford the cinchonidine salt (5), yield 0.88 g (11%), m.p. 149-150°C, $[\alpha]_D^{25}$ = -23.5° (c = 1.00%, methanol).

The diasteretopic purities of salts (4) and (5) were measured as >99.5% by $^1$H - n.m.r. in $C_6D_6$ solution. The $^{13}$C satellites of the dihydropyridine 4-position proton were used as internal standards as described in the Journal of Medicinal Chemistry 1983, 48, 79.

### (B) R-(+)-2-(2-Azidoethoxymethyl)-4-(2-chlorophenyl)-5-methoxycarbonyl-6-methyl-1,4-dihydropyridine-3-carboxylic acid (6)

A suspension of 2-(2-azidoethoxymethyl)-4-(2-chlorophenyl)-5-methoxycarbonyl-6-methyl-1,4-dihydropyridine-3-carboxylic acid, cinchonidine salt (4) ($[\alpha]_D^{25}$ = -78.9°, 3.0 g, 4.3 mmol) in ethyl acetate (50 ml) was washed with 2M hydrochloric acid (50 ml) and then water (50 ml). The organic layer was dried ($MgSO_4$), filtered and evaporated to give a white solid which was recrystallised from ethanol to afford (6), yield 1.6 g (92%), m.p. 134-145°C (with decarboxylation), $[\alpha]_D^{25}$ = +34.7° (c = 0.98% in methanol).

### (C) S-(-)-2-(2-Azidoethoxymethyl)-4-(2-chlorophenyl)-5-methoxycarbonyl-6-methyl-1,4-dihydropyridine-3-carboxylic acid (7)

By the use of an identical procedure to that described in (B) above for the preparation of (6), the cinchonidine salt (5) (0.75 g) was converted to the dihydropyridine 3-carboxylic acid (7), yield 0.37 g (86%), m.p. 137-138° (with decarboxylation), $[\alpha]_D^{25}$ = -36.1° (c = 1.05%, methanol).

### (D) S-(+)-2-(2-Azidoethoxymethyl)-4-(2-chlorophenyl)-3-ethoxycarbonyl-5-methoxycarbonyl-1,4-dihydropyridine (8)

Carbonyl diimidazole (660 mg, 4.1 mmol) was added portionwise to a solution of R-(+)-2-(2-azidoethoxymethyl)-4-(2-chlorophenyl)-5-methoxycarbonyl-1,4-dihydropyridine-3-carboxylic acid (6) (1.5 g, 3.69 mmol) in methylene chloride maintained at 0°C with cooling. After 15 minutes, the cooling was removed and stirring was continued at room temperature for a further 30 minutes. The mixture was then evaporated to

dryness and residue taken up in ethanolic sodium ethoxide (30 ml ethanol containing 90 mg, 3.9 mmol sodium). The resultant orange solution was stirred at room temperature until the colour had dissipated (about 45 minutes). The reaction mixture was diluted with ethyl acetate, washed with 2M hydrochloric acid and then with water, dried ($MgSO_4$), filtered and evaporated to give a colourless oil which crystallised from ethanol to afford (8), yield 1.23 g (77%), m. p. 85 °C, $[\alpha]_D^{25}$ = +34.2° (c = 1.05%, methanol).

(E) R-(-)-(2-Azidoethoxymethyl)-4-(2-chlorophenyl)-3-ethoxycarbonyl-5-methoxycarbonyl-1,4-dihydropyridine (9)

By the use of an identical procedure to that described in (D) above for the preparation of (8), the S-(-)carboxylic acid (7) (500 mg, 1.23 mmol) was converted to the ethyl ester (9), yield 420 mg (77%), m.p. 86 °C, $[\alpha]_D^{25}$ = -36.7° (c = 1.04%, methanol).

(F) S-(+)-2-(2-Azidoethoxymethyl)-4-(2-chlorophenyl)-3-ethoxycarbonyl-5-methoxycarbonyl-1,4-dihydropyridine maleate (10)

A solution of S-(+)-2-(2-azidoethoxymethyl)-4-(2-chlorophenyl)-3-ethoxycarbonyl-5-methoxycarbonyl-1,4-dihydropyridine (8) (1.2 g, 2.76 mmol) in ethanol containing 5% Pd/$CaCO_3$ was hydrogenated at 15 p.s.i. (103.4 kPa) for 3 hours. The hydrogenation mixture was filtered and the filtrate was evaporated to dryness. The residue was taken up in ethyl acetate (15 ml) and a saturated solution of maleic acid in ethyl acetate (15 ml) was added, the resultant precipitate being collected by filtration to afford the maleate (I0), yield 1.38 g (95%), m.p. I72-173 °C, $[\alpha]_D^{25}$ = +27.3° (c + 1.09%, methanol).

(G) R-(-)-2-(2-Aminoethoxymethyl)-4-(2-chlorophenyl)-3-ethoxycarbonyl-5-methoxycarbonyl-1,4-dihydropyridine maleate (11)

By the use of an identical procedure to that described in (F) above for the preparation of (10), the R-(-) azide (9) (397 mg, 0.91 mmol) was converted to the maleate (11), yield 300 mg (77%), m.p. I70-I72 °C, $[\alpha]_D^{25}$ = -26.3° (c = 1.03%, methanol).

| Analysis Figures (%) | | | | | | | |
|---|---|---|---|---|---|---|---|
| Compound | Formula | Found | | | Calculated | | |
| | | C | H | N | C | H | N |
| (4) | $C_{18}H_{19}ClN_4O_5.C_{19}H_{22}N_2O$ | 63.4 | 5.85 | 12.2 | 63.4 | 5.9 | 12.0 |
| (5) | $C_{18}H_{19}ClN_4O_5.C_{19}H_{22}N_2O$ | 62.9 | 5.7 | 11.8 | 63.4 | 5.9 | 12.0 |
| (6) | $C_{18}H_{19}ClN_4O_5$ | 53.0 | 4.8 | 13.5 | 53.1 | 4.7 | 13.8 |
| (7) | $C_{18}H_{19}ClN_4O_5$ | 53.1 | 4.75 | 13.5 | 53.1 | 4.7 | 13.8 |
| (8) | $C_{20}H_{23}ClN_4O_5$ | 55.25 | 5.4 | 12.9 | 55.2 | 5.3 | 12.9 |
| (9) | $C_{20}H_{23}ClN_4O_5$ | 55.25 | 5.1 | 12.7 | 55.2 | 5.3 | 12.9 |
| (10) | $C_{20}H_{25}ClN_2O_5.C_4H_4O_4$ | 55.2 | 5.8 | 5.2 | 54.9 | 5.6 | 5.3 |
| (11) | $C_{20}H_{25}ClN_2O_5.C_4H_4O_4$ | 54.5 | 5.4 | 5.1 | 54.9 | 5.6 | 5.3 |

Formulae

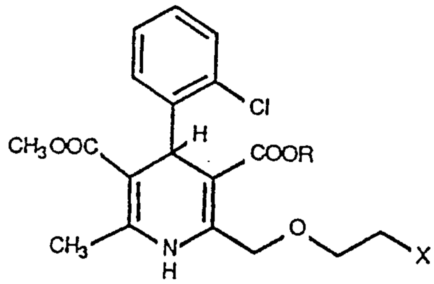

1 R = $CH_2CH_3$ X = $NH_2$

2 R = $CH_2CH(OCH_3)Ph$ (S) X = $N_3$

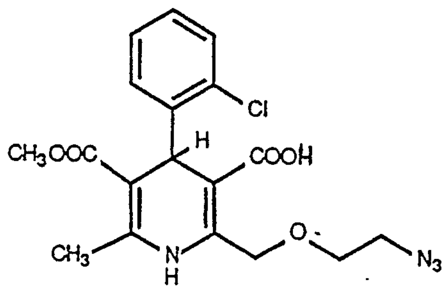

3

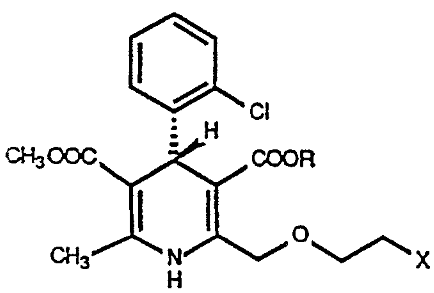

4 R = ( cinchonidine . H ) X = $N_3$

6 R = H X = $N_3$

8 R = $CH_2CH_3$ X = $N_3$

10 R = $CH_2CH_3$ X = $NH_2$
( S-(+) Amlodipine )

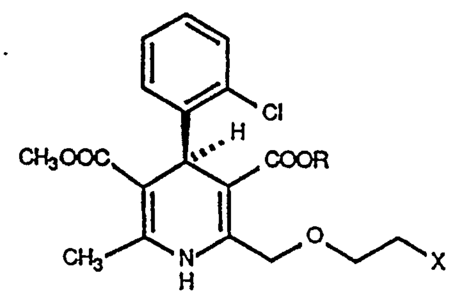

5 R = ( cinchonidine . H ) X = $N_3$

7 R = H X = $N_3$

9 R = $CH_2CH_3$ X = $N_3$

11 R = $CH_2CH_3$ X = $NH_2$
( R-(-) Amlodipine )

**Claims**

1. A salt of 2-(2-azidoethoxymethyl)-4-(2-chlorophenyl) -5-methoxycarbonyl-6-methyl-1,4-dihydropyridine-3-carboxylic acid with cinchonidine.

2. The R- and S- forms of 2-(2-azidoethoxymethyl)-4-(2-chlorophenyl)-5-methoxycarbonyl-6-methyl-1,4-dihydropyridine-3-carboxylic acid.

3. A method of preparing the R- or S- form of amlodipine, or of an acid addition salt thereof, which comprises

(i) forming a salt of R,S-2-(2-azidoethoxymethyl)-4-(2-chlorophenyl)-5-methoxycarbonyl-6-methyl-1,4-dihydropyridine-3-carboxylic acid with cinchonidine and separating said salt into its diastereomeric forms by fractional crystallisation;

(ii) converting said separated salts into the free acids by acidification;

(iii)esterifying the free carboxy groups to ethoxycarbonyl; and

(iv) reducing the azido groups to amino, thus producing separated R-(-)- and S-(+)- amlodipine, and isolating the separated forms of amlodipine as such or in acid addition salt form.

Claims for the following Contracting State: ES

1. A method of preparing the R- or S- form of amlodipine, or of an acid addition salt thereof, which comprises

(i) forming a salt of R,S-2-(2-azidoethoxymethyl)-4-(2-chlorophenyl)-5-methoxycarbonyl-6-methyl-1,4-dihydropyridine-3-carboxylic acid with cinchonidine and separating said salt into its diastereomeric forms by fractional crystallisation;

(ii) converting said separated salts into the free acids by acidification;

(iii) esterifying the free carboxy groups to ethoxycarbonyl; and

(iv) reducing the azido groups to amino, thus producing separated R-(-)- and S-(+)- amlodipine, and isolating the separated forms of amlodipine as such or in acid addition salt form.